(19) Europäisches Patentamt
European Patent Office
Office européen des brevets

(11) Publication number: 0 078 615 B1

(12) EUROPEAN PATENT SPECIFICATION

(45) Date of publication of patent specification: 03.07.85

(21) Application number: 82305347.5

(22) Date of filing: 07.10.82

(51) Int. Cl.[4]: C 07 C 43/13, C 07 C 43/10, C 07 C 49/175, C 07 C 41/01, C 07 C 41/28, C 07 C 45/49, B 01 J 31/20

(54) **Process for preparing glycol ethers.**

(30) Priority: 29.10.81 US 316192
29.10.81 US 316193

(43) Date of publication of application:
11.05.83 Bulletin 83/19

(45) Publication of the grant of the patent:
03.07.85 Bulletin 85/27

(84) Designated Contracting States:
BE DE FR GB IT NL SE

(58) References cited:
DE-A-2 655 406

(73) Proprietor: TEXACO DEVELOPMENT CORPORATION
2000 Westchester Avenue
White Plains New York 10650 (US)

(72) Inventor: Knifton, John Frederick
10900 Catskill Trail
Austin Texas 78750 (US)

(74) Representative: Brock, Peter William et al
Michael Burnside & Partners 2 Serjeants' Inn
Fleet Street
London EC4Y 1HL (GB)

EP 0 078 615 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for preparing glycol ethers. More particularly this invention relates to a process for preparing monoalkyl ethers of ethylene glycol and propylene glycol by the reaction of synthesis gas (i.e. a mixture of hydrogen and carbon monoxide) with, (a) formaldehyde, acetaldehyde, or an acetal thereof, and (b) an alcohol. A cobalt catalyst, promoted by a compound of tin or germanium is employed.

There is an ever increasing need for a wide variety of glycol monoalkyl ethers of different carbon numbers and structures which have become important articles of commerce. Such ethers are employed in a wide variety of application as solvents, reaction media, etc. In conventional processes, an olefin oxide such as ethylene oxide or propylene oxide, is first prepared from an olefin and reacted with suitable alcohol. Since the cost of materials derived from petroleum sources has been rising rapidly, research efforts are now being made to find a new process for producing these glycol ethers which does not utilize an olefin as a starting material. One of the newer methods for the preparation of glycol monoalkylethers, in which an acetal is reacted with carbon monoxide and hydrogen in the presence of a cobalt carbonyl catalyst, is described in German Patents 875,802 and 890,945. This process suffers from several disadvantages, including a low selectivity of the glycol ether, and decomposition of the carbonyl catalyst during recovery of the product from the reaction mixture.

In US—A—4071568 (corresponding to DE—A—2655406) a process for making glycol monoalkyl ethers is disclosed in which the catalyst utilized is cobalt carbonyl combined with a trivalent organic phosphorus compound such as tri-n-butylphosphine which is reported to give better selectivity. Other processes for preparing glycol monoalkyl ethers are described in U.S. 4,062,898 and in German 2,741,589.

One of the objects of this invention is to provide a novel process for preparing ethylene and propylene glycol monoalkyl ethers by means of a unique catalyst system in which the feedstock utilized comprises formaldehyde, acetaldehyde or an acetal thereof, an alcohol and synthesis gas.

This invention provides a process for preparing ethylene or propylene glycol ethers by reacting hydrogen, carbon monoxide and an aldehyde or acetal of the formula

$$RCHO \text{ or } RCH(OR')_2$$

wherein R is hydrogen or methyl and R' is $C_{1-10}$ alkyl, in the presence of a cobalt-containing catalyst and an alcohol of the formula

$$R'OH$$

wherein R' is as defined above, at a pressure of at least 35 bars and a temperature of 50 to 350°C, in which process the reaction is conducted in the presence of a tin-containing compound or a germanium-containing compound as catalyst promoter.

In one embodiment of the invention, formaldehyde or an acetal thereof or a formaldehyde-releasing compound is reacted at a temperature of from 50 to 300°C and a pressure from 70 to 350 bars to give a product containing ethylene glycol ethers.

In this embodiment, formaldehyde or its acetal react as follows:

i) $HCHO+R'OH+CO+2H_2 \rightarrow HO \cdot CH_2CH_2 \cdot OR'+H_2O$
ii) $(R'O)_2CH_2+CO+2H_2 \rightarrow HO \cdot CH_2CH_2 \cdot OR'+R'OH$

In another embodiment of the invention, acetaldehyde or an acetal thereof is reacted at a temperature of 50 to 350°C and a pressure of 70 to 700 bars to give a product containing propylene glycol ether of the formulae

$$\begin{array}{ccc} CH_2OR' & & CH_2OH \\ | & \text{and} & | \\ CHOH & & CHOR' \\ | & & | \\ CH_3 & & CH_3 \end{array}$$

generally an alkoxyacetone of the formula

$$CH_3—CO—CH_2OR'$$

is obtained in association with the propylene glycol ethers. In these formulae R' has the meaning given above.

In this latter embodiment, acetaldehyde and an alcohol react with synthesis gas as follows:

iii) 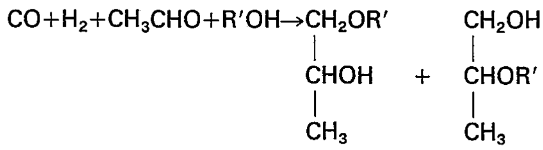

iv) 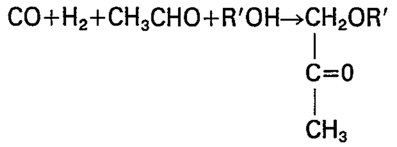

Under certain operating conditions, alkoxyacetones are formed as the major products in this embodiment of the process according to the reaction scheme of equation (iv) above.

A high degree of selectivity is exhibited by the reaction of the process of this invention. For example, when acetaldehyde and ethanol are reacted with synthesis gas substantial yields of the propylene glycol monoethyl ethers and the ethoxyacetone are obtained.

In carrying out the reaction of this invention selectively to produce high yields of the desired ethylene glycol ethers or the desired propylene glycol monoalkylethers and/or alkoxyacetones it is necessary to supply sufficient carbon monoxide, hydrogen, aldehyde or acetal and alcohol to at least satisfy the stoichiometry of equations (i), (ii), (iii) and/or (iv) above although an excess of one or more of the reactants over the stoichiometric amounts may be present.

Catalysts that are suitable for use in the practice of this invention contain cobalt. The cobalt-containing compounds may be chosen from a wide variety of organic or inorganic compounds, complexes, or as will be shown and illustrated below. It is only necessary that the catalyst precursor actually employed contain said metal in any of its ionic states. The actual catalytically active species is then believed to comprise cobalt in complex combination with one or more ligands and with carbon monoxide and hydrogen. The most effective catalyst is achieved where the cobalt hydrocarbonyl species is solubilized in the alcohol coreactant employed.

The cobalt-containing catalyst precursors may take many different forms. For instance, the cobalt may be added to the reaction mixture in an oxide form, as in the case of, for example, cobalt(II) oxide ($CoO$), or cobalt(II, III) oxide ($Co_3O_4$). Alternatively, it may be added as the salt of a mineral acid, as in the case of cobalt(II) chloride ($CoCl_2$), cobalt(II), chloride hydrate ($CoCl_2 \cdot 6H_2O$), cobalt(II) bromide ($CoBr_2$), cobalt(II) iodide ($CoI_2$) and cobalt(II) nitrate hydrate ($Co(NO_3)_2 \cdot 6H_2O$), or as the salt of a suitable organic carboxylic acid, for example, cobalt(II) formate, cobalt(II) acetate, cobalt(II) propionate, cobalt naphthenate, and cobalt acetylacetonate. The cobalt may also be added to the reaction zone as a carbonyl or hydrocarbonyl derivative. Here, suitable examples include dicobalt octacarbonyl ($Co_2(CO)_8$), cobalt hydrocarbonyl ($HCo(CO)_4$) and substituted carbonyl species such as the triphenylphosphine cobalt tricarbonyl dimer.

Preferred cobalt-containing compounds include oxides of cobalt, cobalt salts of a mineral acid, cobalt salts of organic carboxylic acids and cobalt carbonyl or hydrocarbonyl derivatives. Among these, particularly preferred are cobalt(II) chloride, cobalt acetylacetonate, cobalt(II) acetate, cobalt(II) propionate, and dicobalt octacarbonyl.

As previously pointed out in the process of this invention the reaction is conducted in the presence of a catalyst comprising a cobalt-containing compound and a tin- or germanium-containing promoter. The cobalt-containing compound employed may be a cobalt carbonyl or a compound capable of forming a cobalt carbonyl under reaction conditions.

The tin-containing promoter compounds which may be utilized with the cobalt-containing compounds in this process may also take many different forms. For instance the tin may be added to the reaction mixture in elemental form, or in the form of a halide, such as stannic chloride, stannous iodide, stannic bromide, or a hydrocarbonyl tin compound such as tetraphenyltin, tetra-n-butyltin, hexamethylditin, tetramethyltin, and dibutyl diphenyltin, or as organohalide tin compound such as trimethyltin chloride, di-t-butyltin dichloride, dimethyltin dichloride, methyltin trichloride, phenyltin trichloride, triethyltin bromide, trimethyltin bromide and tributyltin bromide, or an organotin hydride such as tributyltin hydride, or an organotin oxide such as dimethyltin oxide and diphenyltin oxide, or a carboxylate such as tin(II) caproate, tributyltin acetate, and tri-n-propyltin acetate, or an oxide such as stannous oxide and stannic oxide.

The preferred tin-containing promoter compounds are the organo-halide tin compounds. Among these, particularly preferred are trimethyltin chloride, tributyltin hydride and tributyltin bromide.

The germanium-containing promoter compounds which may be utilized with the cobalt-containing compounds in this process may also take many different forms. For instance, the germanium may be added to the reaction mentioned in the form of a halide, such as germanium tetrachloride, germanium diiodide and germanium tetrabromide, or as a hydrocarbylgermanium compound such as tetra-n-butylgermane, tetraethylgermane, tetraphenylgermane and tetramethylgermane, or an organohalide germanium compound such as diphenylgermanium chloride, methylgermanium trichloride, phenylgermanium trichloride, tri-n-butylgermanium iodide, triethylgermanium chloride, triethylgermanium

iodide, trimethylgermanium chloride, triphenyl-germanium bromide and triphenylgermanium chloride or an organogermanium hydride, such as triphenylgermanium hydride, or an organogermanium oxide or carboxylate such as triphenylgermanium acetate, or a germanium alkoxide such as germanium butoxide, germanium ethoxide and germanium methoxide.

The preferred germanium-containing promoter compounds are the organo-halide germanium compounds, the hydrocarbylgermanium compounds and the organogermanium hydrides. Among these, particularly preferred are triphenylgermanium bromide, triphenylgermanium hydride, trimethylgermanium chloride, triphenylgermanium chloride, trimethylgermanium bromide, triethylgernamnium chloride, tetraphenylgermane and tetramethylgermane.

The amount of the tin-containing or germanium-containing compound employed can be varied widely in this process and is generally in the range of 0.01 to 100 and preferably from 0.1 to 5 gram moles per gram atom of cobalt.

The quantity of cobalt catalyst employed in the instant invention is not critical and may vary over a wide range. In general, the novel process is desirably conducted in the presence of a catalytically effective quantity of one or more of the active cobalt species together with one or more of the tin- or germanium-containing promoters which gives the desired products in reasonable yields. The reaction proceeds when employed as little as $1\times10^{-6}$ weight percent and even lesser amount of cobalt, basis the total weight of the reaction mixture. The upper concentration is dictated by a variety of factors including catalyst cost, partial pressures of carbon monoxide and hydrogen and operating temperature.

A cobalt concentration of from $1\times10^{-5}$ to 10 weight percent cobalt, based on the total weight of reaction mixture, is generally desirable in the practice of this invention.

Alcohols useful as starting materials in the process of this invention (see equations (i) to (iv) above) have the formula:

$$R'OH$$

where R' is an alkyl radical of from 1 to 10 carbon atoms. Suitable alcohols include methanol, ethanol, propanol, butanol, heptanol, decanol, and isomers thereof.

Formaldehyde acetals which may be utilized in the process of this invention include compounds of formula:

$$(R'O)_2CH_2,$$

where R' is alkyl of from 1 to 10 carbon atoms as exemplified by methyl, ethyl, butyl, hexyl, nonyl, and isomers thereof. The preferred acetals include dimethoxymethane, di-n-butoxymethane, diethoxymethane, and dipropoxymethane.

Acetals of acetaldehyde which may be utilized in the process of this invention include compounds of the formula:

$$(R'O)_2CH_2CH_3$$

where R' has the meaning given above. The preferred acetals include 1,1-dimethoxyethane, 1,2-diethoxyethane (commonly known as acetal) and 1,1-di-n-propyloxyethane.

The acetal or aldehyde are used in combination with an alcohol. The ratio of alcohol to aldehyde is not critical but is usually in the range of 0.01 to 100 moles of alcohol per mole of aldehyde (or acetal).

The relative amounts of carbon monoxide and hydrogen which may be initially present in the syngas mixture are also variable, and these amounts may be varied over a wide range. In general, the mole ratio of $CO:H_2$ is in the range from 20:1 up to 1:20, preferably from 5:1 to 1:5, although ratios outside these ranges may also be employed. Particularly in continuous operations, but also in batch experiments, the carbon monoxide-hydrogen gaseous mixtures may also be used in conjunction with up to 50% by volume of one or more other gases. These other gases may include one or more inert gases such as nitrogen, argon and neon, or they may include gases thay may, or may not, undergo reaction under CO hydrogenation conditions, such as carbon dioxide, hydrocarbons such as methane, ethane and propane, ethers such as dimethyl ether, methylethyl ether and diethyl ether.

The temperature range which can usefully be employed in these syntheses is a further variable, dependent upon other experimental factors, including the choice of the alcohol, the pressure, and the concentration and choice of particular species of the cobalt-containing compound and the promoter compounds among other things. The range of operability is from 50 to 350°C, although with formaldehyde or an acetal thereof, the preferred maximum temperature is 300°C. A narrower range of 100 to 250°C represents the most preferred temperature range.

Superatmospheric pressures of 35 bars or greater leads to substantial yields of desirable glycol ethers by the process of this invention. Preferred operating ranges are from 70 to 350 bars when using formaldehyde (or its acetal), and 70 to 700 bars when using acetaldehyde (or its acetal), although pressures above 700 bars also provide useful yields of the desired products. The pressures referred to here represent the total pressure generated by all the reactants, although they are substantially due to the carbon monoxide and hydrogen fractions in these examples.

As far as can be determined, without limiting the invention thereby, the one-step process disclosed herein leads primarily to the formation of ethylene glycol monoalkyl ethers or propylene glycol monoalkyl ethers and alkoxyacetone products. Ethylene glycol dialkyl ether products are also formed, and in the case, for example, where formaldehyde and n-butanol are the co-reactants, the principal products are ethylene glycol monobutyl ether and ethylene glycol dibutyl ether. Diethylene glycol monobutyl ether, methanol, butyl formate and water are also detected in the liquid product fraction. The propylene monoalkyl ethers may be generated in this process in two isomeric forms, i.e., the propylene glycol α-monoalkyl ethers and propylene glycol β-monoalkyl ethers which have the general structures I and II respectively, where in this case R′ is an alkyl radical of from 1 to 10 carbon atoms.

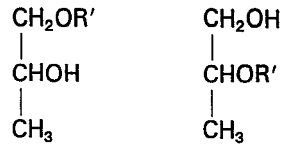

I II

In the case where acetaldehyde and ethanol are the coreactants, the principal products are the propylene glycol monoethyl ethers and ethoxyacetone. By-products such as water, ethanol and diethyl ether are also detected in the liquid product fraction.

The novel process of this invention can be conducted in a batch, semicontinuous or continuous fashion. The catalyst may be initially introduced into the reaction zone batchwise, or it may be continuously or intermittently introduced into such a zone during the course of the synthesis reaction. Operating conditions can be adjusted to optimize the formation of the desired ether products, and said material may be recovered by methods well known in the art, such as distillation, fractionation, extraction and the like. A fraction rich in the cobalt-containing compound and the promoter compound may then be recycled to the reaction zone, if desired, and additional products generated.

The products formed by the process of this invention have been identified in this work by one or more of the following analytical procedure, viz, gas-liquid phase chromatograph (glc), infrared (ir), mass spectrometry, nuclear magnetic resonance (nmr) and elemental analysis, or a combination of these techniques. Analyses have, for the most part, been by parts in weight, all temperatures are in degrees centigrade and all pressures in bars.

The following Examples which illustrate various embodiments of the invention are to be considered not limitative.

Example 1

To a 450 ml glass-lined, pressure reactor was charged a mixture of dicobalt octacarbonyl (2 mmole Co.), triphenylgermanium bromide (2 mmole) and paraformaldehyde (0.1 mole) in 37.1 g of n-butanol (0.5 mole). The mixture was flushed with nitrogen, the reactor sealed, flushed with $CO/H_2$ (1:1 molar ratio), pressured to 187.2 bars with 2:1 molar ratio syngas ($H_2/CO$) and then heated to 160°C with agitation. After four hours, the reactor was allowed to cool the gas pressure (173.4 bars) noted, the excess gas sampled and vented, and the deep red liquid product (44.5 g) recovered.

Analysis (glc and Karl Fischer titration) of the liquid product showed it to contain:

12.2 wt. % ethylene glycol monobutyl ether
1.4 wt % ethylene glycol dibutyl ether
1.2 wt. % water
71.4 wt. % unreacted n-butanol.

Estimated yield of ethylene glycol monobutyl ether (basis formaldehyde charged) was 46 mole percent. Total glycol monobutyl plus dibutyl ether yield was 50 mole percent.

Analysis of the liquid product by atomic adsorption showed it to contain >98% of the cobalt originally charged. There was no solid product phase.

Typical off-gas samples showed the presence of:

58% hydrogen
37% carbon monoxide
3.5% carbon dioxide

The glycol monobutyl ethers were recovered from the crude liquid product by fractional distillation in vacuo.

Example 2

To a 450 ml glass-lined pressure reactor was charged a mixture of dicobalt octacarbonyl (2 mmole Co), triphenylgermanium bromide (2.0 mmoles), dibutoxymethane (0.1 mole) in 22.2 g of n-butanol (0.3 mole). The mixture was flushed with nitrogen, the reactor sealed, flushed with $CO/H_2$ (1:2 molar), was pressured to 187.2 bars with 1:2 molar syngas ($CO/H_2$) and then heated to 160°C with agitation.

After four hours, the reactor was allowed to cool, the gas pressure (159.6 bars) was noted, the excess gas sampled and vented, and the deep reddish-brown liquid products (43.0 g) recovered.

Analysis (glc and Karl Fisher titration) of the liquid product showed it to contain:

11.6 wt. % ethylene glycol monobutyl ether
3.5 wt. % ethylene glycol dibutyl ether
1.7 wt. % water
69.2 wt. % butanol.

Estimated yield of ethylene glycol monobutyl ether (basis dibutoxymethane charged) was 41 mole percent. Total glycol monobutyl plus dibutyl ether yield was 50 mole percent.

Analysis of the liquid product by atomic adsorption showed it to contain 97% of the cobalt originally charged. There was no solid product phase.

Typical off-gas samples showed the presence of:

58% hydrogen
34% carbon monoxide
1.9% carbon dioxide.

Example 3

Following the procedure of Examples 1 and 2, the reactor was charged with a mixture of dicobalt octacarbonyl (2 mole Co), triphenylgermanium bromide (2 mmole), dimethoxymethane (0.1 mole) in 9.6 g of methanol (0.3 mole). The mixture was flushed with nitrogen, the reactor sealed, flushed with $CO/H_2$ (1:2 molar, pressured to 187.2 bars with 1:2 molar syngas ($CO/H_2$) and then heated with agitation to 200°C. After four hours the reactor was allowed to cool, the gas pressure (169.9 bars) noted, the excess gas sampled and vented, and the black liquid product (18.3 g) recovered.

Analysis of the liquid product showed it to contain:

9.8 wt.% ethylene glycol monomethyl ether
3.2 wt. % ethylene glycol dimethyl ether
8.9 wt. % water
9.1 wt. % dimethoxymethane
60.5 wt. % methanol.

Typical off-gas samples showed the presence of:

54% hydrogen
32% carbon monoxide
1.1% carbon dioxide
0.5% methane.

Examples 4—18

A number of addition Examples were carried out using the procedure of Example 1 in which a variety of tin- and germanium-containing promoters were employed along with cobalt octacarbonyl catalyst. Data relating to these Examples are shown in Tables 1 and 2 which follows. Specifically:

a) Examples 4 to 9 illustrate the effectiveness of dicobalt octacarbonyl coupled with various germanium promoters, including triphenylgermanium hydride, tetraphenylgermane, tetraethylgermane, triphenylgermanium chloride, trimethylgermanium bromide and triethylgermanium chloride.

b) Examples 10 to 18 demonstrate the effective use of dicobalt octacarbonyl coupled with various tin-containing promoters, including tributyltin bromide, tributyltin chloride and trimethyltin chloride.

TABLE 1[a,b]
Liquid product composition (wt. %)[c]

| Example | Catalyst composition | $H_2O$ | MeOH | BuOH | EGMBE | EGDBE | EG | EGMBE[d] yield (mole %) |
|---|---|---|---|---|---|---|---|---|
| 4 | $Co_2(CO)_8$—$Ph_3GeCl$ | 4.9 | 0.3 | 72.4 | 12.2 | 1.4 | 0.2 | 45 |
| 5 | $Co_2(CO)_8$—$Me_3GeBr$ | 5.1 | 0.3 | 73.8 | 11.3 | 1.5 | 0.2 | 42 |
| 6 | $Co_2(CO)_8$—$Et_3GeCl$ | 4.9 | 0.3 | 72.9 | 12.3 | 1.4 | 0.2 | 46 |
| 7 | $Co_2(CO)_8$—$Ph_4Ge$ | 4.5 | 0.4 | 74.2 | 14.0 | 1.0 | 0.5 | 53 |
| 8 | $Co_2(CO)_8$—$Et_4Ge$ | 5.1 | 0.3 | 70.9 | 13.2 | 1.6 | 0.3 | 50 |
| 9 | $Co_2(CO)_8$—$Ph_3GeH$ | 4.7 | 0.4 | 72.6 | 13.0 | 1.4 | 0.3 | 47 |

[a]Run Charge: Co, 2 mmoles; Co/Ge=1/1; BuOH, 0.5 mole; HCHO, 0.1 mole.
[b]Run Conditions: 160°C., initial 187.2 bars of $CO/H_2$ (1:2 molar), 4 hours.
[c]Designations: EGMBE, Ethylene glycol monobutyl ether; EGDBE, ethylene glycol dibutyl ether;
[d]EGMBE yield basis formaldehyde charged.

TABLE 2[a,b]
Liquid product compositions (wt.%)[c]

| Example | Catalyst composition | $H_2O$ | MeOH | BuOH | EGMBE | EGDBE | EG | EGMBE[d] yield (mole %) |
|---|---|---|---|---|---|---|---|---|
| 10 | $Co_2(CO)_8$—$Bu_3SnBr$ | 4.6 | 0.4 | 76.3 | 8.2 | 1.1 | 0.2 | 29 |
| 11 | $Co_2(CO)_8$—$Bu_3SnH$ | 1.7 | 0.9 | 93.0 | <0.1 | 0.3 | 0.4 | <2 |
| 12 | $Co_2(CO)_8$—$Bu_3SnOAc$ | 2.4 | 0.8 | 87.4 | 0.3 | 0.6 | 0.2 | <2 |
| 13 | $Co_2(CO)_8$—$Bu_3SnCl$ | 4.8 | 0.3 | 76.4 | 9.9 | 0.7 | 1.5 | 37 |
| 14 | $Co_2(CO)_8$—$Me_3SnCl$ | 4.6 | 0.3 | 73.1 | 8.8 | 0.9 | 0.2 | 33 |
| 15 | $Co_2(CO)_8$—$Ph_3SnCl$ | 3.8 | 0.6 | 85.2 | 0.9 | 0.3 | 0.3 | 3 |
| 16 | $Co_2(CO)_8$—$Ph_4Sn$ | 3.8 | 0.3 | 83.1 | 1.3 | 0.3 | 0.3 | 5 |
| 17 | $Co_2(CO)_8$—$SnCl_4$ | 4.4 | 0.4 | 82.4 | 1.4 | 0.8 | 0.2 | 5 |
| 18 | $Co_2(CO)_8$—$SnCl_2$ | 4.4 | 0.4 | 87.7 | 1.8 | 0.8 | 0.1 | 6 |

[a]Run Charge: Co, 2 mmoles; Co/Sn=1/1; BuOH, 0.5 mole; HCHO, 0.1 mole.
[b]Run Conditions: 160°C; initial 187.2 bars of $Co/H_2$ (1:2 molar); 4 hours.
[c]Designations as per Table 1
[d]EGMBE yield basis formaldehyde charged.

Example 19

Following the procedure of Example 1, the reactor was charged with a mixture of dicobalt octacarbonyl (2 mmole Co), triphenylgermanium bromide (2 mmole) and paraformaldehyde (0.1 mole) in 16.0 g of methanol (0.5 mole). The mixture was flushed with $CO/H_2$, the reactor sealed, pressured to 187.2 bars with $CO/H_2$ (1:2 molar) and heated with agitation to 180°C. After four hours, the reactor was allowed to cool, the excess gas vented and the liquid product (23.4 g) recovered.

Analysis of the liquid product showed it to contain 8.4 wt. % of ethylene glycol monomethyl ether and 0.7 wt. % of ethylene glycol dimethyl ether. Cobalt recovery in solution was >98% of that originally charged, there was no solid product phase.

Example 20

Following the general procedure of Example 1, the 450 ml glass-lined pressure reactor was charged with 0.1 mole of paraformaldehyde, dicobalt octacarbonyl (2.0 mmole Co), triphenylgermanium bromide (2.0 mmole) and 0.5 mole of *n*-butanol. After flushing with syngas ($CO/H_2$ mixture), the reactor was pressured to 187.2 bars with a gaseous mixture containing 2 moles of hydrogen per mole of carbon monoxide, and heated to 160°C with agitation. After 4 hours at temperature, the reactor was cooled and vented and the clear red liquid product (44.5 g) recovered and analyzed by glc and Karl Fischer titration. There were no residual solids at this state.

The product liquid was distilled *in vacuo*, and ethylene glycol monobutyl ether was recovered as a distillate fraction at 48—52°C (1 mm Hg pressure). The residual catalyst remained behind as a deep-red colored liquid (3.6 g) and this residual catalyst liquid was returned to the 450 ml glass-lined reactor by washing with additional n-butanol (0.5 mole). Fresh paraformaldehyde (0.1 mole) was also added at this stage, the reactor sealed, flushed with syngas, pressured to 187.2 bars with $CO/H_2$ (1:2 molar) and heated to 160°C with agitation for 4 hours. In this manner the synthesis of ethylene glycol monobutyl ether was repeated successfully, and the later recovered from the crude liquid product (44.6 g) by vacuum distillation as outlined above.

The residual catalyst solution (5.1 g) from this second cycle was again returned to the reactor for further glycol ether synthesis. The yields of glycol monobutyl ether (basis paraformaldehyde charged) for this three cycle experiment are shown in Table 3.

TABLE 3

Ethylene glycol butyl ethers from syngas-catalyst recycling

| Example | Number of catalyst cycles | Yield of $BuOCH_2CH_2OH$(%)[a] |
|---|---|---|
| 20 | 1 | 46 |
| | 2 | 47 |
| | 3 | 45 |

[a]Yield basis paraformaldehyde charged (0.1 mole) at the start of each catalyst cycle.

Example 21

A 450 ml glass-lined pressure reactor was charged with a mixture of dicobalt octacarbonyl (20 mmole Co), triphenylgermanium hydride (2.0 mmole) and 1,1-diethoxyethane, i.e., acetal (0.1 mole) in 23.0 g of ethanol (0.5 mole). The mixture was charged under a nitrogen atmosphere, the reactor sealed, flushed with $CO/H_2$ (1:2 molar), pressured to 311.3 bars with 1:2 molar syngas ($CO/H_2$) and then heated to 180°C with agitation.

After carbonylation the reactor was cooled, the gas pressure noted (277.9 bars), the excess gas sampled and vented, and the dark brown liquid product (40.5 g) recovered.

Analysis of the liquid product by glc and Karl Fischer titration showed it to contain:

- 4.9 % propylene glycol α-monoethyl ether
- 3.0% propylene glycol β-monoethyl ether
- 0.7% 1-ethoxyacetone
- 4.5% water
- 7.8% diethylether
- 66.6% ethanol
- 1.4% unreacted acetal

Estimated yield of propylene glycol monoethyl ethers (Basis acetal charged) =31 mole %.

Yield of propylene glycol monoethyl ethers+1-ethoxyacetone (basis acetal converted) =35 mole %.

Typical off-gas samples showed the presence of:

- 66% hydrogen
- 27% carbon monoxide
- 3.4% carbon dioxide.

The propylene glycol monoethyl ethers were recovered from the crude liquid product by fractional distillation in vacuo.

Example 22

Following the operating procedures of Example 21, the reactor was charged with a mixture of dicobalt octacarbonyl (2 mmole Co), triethylgermanium chloride (2.0 mmole Co), acetal (0.05 mole) and 13.80 g of ethanol (0.3 mole). After pressuring to 311.3 bars with 1:2 molar syngas ($CO/H_2$), the reactor was heated to 180°C with agitation.

After 8 hours, the reactor was cooled, the gas pressure noted (256.1 bars), the excess gas sampled and vented and the red liquid product (22.3 g) recovered.

Analysis of the liquid product by glc and Karl Fischer titration showed it to contain:

6.2% propylene glycol α-monoethyl ether
4.0% propylene glycol β-monoethyl ether
0.2% ethoxyacetone
4.9% water
1.5% diethyl ether
69.8% ethanol
0.2% unreacted acetal

Estimated yield of propylene glycol monoethyl ethers (basis acetal charged)
=44 mole %

Yield of propylene glycol monoethylethers+ethoxyacetone (basis acetal converted)
=45 mole %

Typical off-gas samples showed the presence of:

55% hydrogen
40% carbon monoxide
37% carbon dioxide

Analysis of the liquid product by atomic absorption showed it to contain 98% of the cobalt originally charged. There was no solid product phase.

Example 23

Following the operating procedures of Example 21, the reactor was charged with a mixture of dicobalt octacarbonyl (2 mmole Co), triphenylgermanium hydride (2.0 mmole), acetaldehyde (0.1 mole) and 37.1 g of n-butanol (0.5 mole). After pressuring to 187.2 bars with 1:2 molar syngas ($CO/H_2$), the reactor was heated to 160°C with agitation.

After four hours, the reactor was cooled, the gas pressure (177.9 bars) noted, the excess gas sampled and vented and the deep-red liquid product (44.2 g) recovered. There was no solid phase.

Analysis of the liquid product by glc and Karl Fischer titration showed it to contain:

6.6% n-butoxypropanone
3.1% propylene glycol monobutyl ethers
5.3% water
79.3% n-butanol
0.2% unreacted acetaldehyde

Typical off-gas samples show the presence of:

56.5% hydrogen
31.6% carbon monoxide
2.2% carbon dioxide

Example 24

Following the operating-procedures of Example 21, the reactor was charged with a mixture of dicobalt octacarbonyl (2 mmole Co), triphenylgermanium hydride (2.0 mmole), acetaldehyde (0.1 mole) and 18.0 g of n-propanol (0.3 mole). After pressuring to 311.3 bars with 2:1 molar syngas ($CO/H_2$), the reactor was heated to 180°C with agitation.

After four hours, the reactor was cooled, the gas pressure note (273.4 bars) the excess gas sampled and vented and the liquid product (24.9 g) recovered.

Analysis of the liquid product by glc and Karl Fischer titration showed it to contain:

4.8% propylene glycol monopropyl ethers
4.2% n-propoxyacetone
4.5% water
66.9% n-propanol
7.7% ethanol
0.5% unreacted acetaldehyde

Typical off-gas samples showed the presence of:

2.9% hydrogen
7.0% carbon monoxide
0.1% carbon dioxide

Analysis of the liquid product showed it to contain >98% of the cobalt originally charged. There was no solid product phase.

Example 25

Following the operating procedure of Example 23, the reactor was charged with a mixture of dicobalt octacarbonyl (2 mmole Co), triphenylgermanium hydride (2.0 mmole), acetaldehyde (0.1 mole) and 37.1 g of n-butanol. After pressuring to 311.3 bars with 1:2 molar syngas ($CO/H_2$) the reactor was heated to 180°C with agitation.

After four hours, the reactor was cooled, the gas pressure noted (287.2 bars), the excess gas sampled and vented and the liquid product (44.1 g) recovered.

Analysis of the liquid product by glc and Karl Fischer titration showed it to contain:

5.6% propylene glycol monobutyl ethers
1.8% n-butoxypropanone
2.8% water
76.9% n-butanol
5.2% ethanol
<0.1% unreacted acetaldehyde

Typical off-gas samples showed the presence of:

5.6% hydrogen
4.1% carbon monoxide
2.0% carbon dioxide

Analysis of the liquid product showed it to contain >98% of the cobalt originally charged. There was no solid process phase.

Examples 26—39

Using the same procedure as in Example 21, a number of additional Examples were carried out in which acetal was reacted with carbon monoxide and hydrogen in the presence of a variety of tin and germanium promoters. Data relating to these Examples are included in Tables 4 and 5 which follow.

It may be noted that:

1) Propylene glycol monoethyl ethers may be generated from synthesis gas, acetal (1,1-diethoxyethane) and ethanol using solubilized cobalt octacarbonyl coupled with a variety of tin and germanium promoters. Suitable promoters include triphenylgermanium bromide, triphenylgermanium hydride, triethylgermanium chloride, tetraethylgermane, trimethylgermanium bromide and tetraphenylgermane.

2) Cobalt recovery in solution was >98% for many of these cobalt catalyst combinations.

3) Syntheses were conducted over the temperature range 160—180°C when employing reaction times of 4 to 18 hours.

TABLE 4

Propylene glycol monoalkyl ethers by reaction of synthesis gas, ethanol and acetal[a]

| Example | Catalyst composition | Aldehyde co-reactant | Time hour | Liquid product composition (wt. %)[b] 1-Et 2-Pro | 2-Et, 1-Pro | 1-EtAcet | Acetal | $H_2O$ | EtOH | Cobalt recovered (%) |
|---|---|---|---|---|---|---|---|---|---|---|
| 26 | $Co_2(CO)_8$—$Ph_3GeBr$ | Acetal | 4 | 1.4 | 1.5 | 8.8 | 1.4 | 2.6 | 70.0 | >98 |
| 27 | $Co_2(CO)_8$—$Ph_3GeH$ | Acetal | 4 | 1.0 | 1.6 | 8.6 | 1.5 | 3.0 | 73.5 | |
| 28 | $Co_2(CO)_8$—$Me_3GeBr$ | Acetal | 4 | 1.7 | 1.6 | 8.4 | 0.7 | 3.5 | 70.6 | >98 |
| 29 | $Co_2(CO)_8$—$Bu_3SnH$ | Acetal | 4 | 0.1 | | 6.7 | 17.6 | 1.4 | 64.6 | >98 |
| 30 | $Co_2(CO)_8$—$Bu_3SnCl$ | Acetal | 4 | 0.6 | | 7.5 | 3.0 | 2.1 | 78.2 | |
| 31 | $Co_2(CO)_8$—$Et_4Ge$[c] | Acetal | 4 | 5.8 | 5.0 | 4.2 | 2.6 | 4.2 | 65.2 | |
| 32 | $Co_2(CO)_8$—$Ph_4Ge$[c] | Acetal | 4 | 4.2 | 3.5 | 4.1 | 2.2 | 3.3 | 73.3 | |
| 33 | $Co_2(CO)_8$—$Et_3GeCl$[c] | Acetal | 4 | 4.7 | 4.8 | 5.7 | 2.2 | 3.6 | 66.4 | >98 |

[a]Run Conditions: Co, 2 mmole; Ge/Sn, 2 mmole; Acetal, 0.1 mole; EtOH, 0.3 mole; 160°C; 187.2 bars ($CO/H_2$, 1:2 Molar).

[b]Abbrev: 1-Et, 2-Pro, 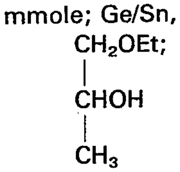 ; 2-Et, 1-Pro, 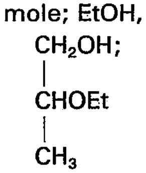 ; 1-EtAcet, 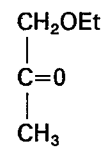

[c]Run Conditions: 180°C, 311.3 bars ($CO/H_2$, 1:2 molar).

TABLE 5

Propylene glycol monoalkyl ethers from synthesis gas, ethanol and acetal[a]

| Example | Catalyst composition | Aldehyde co-reactant | Time hour | Liquid product composition (wt.%)[b] | | | | | | | Cobalt recovered (%) |
|---|---|---|---|---|---|---|---|---|---|---|---|
| | | | | 1-Et, 2-Pro | 2-Et, 1-Pro | 1-EtAcet | Acetal | $H_2O$ | EtOH | $Et_2O$ | |
| 34 | $Co_2(CO)_8$—$Et_3GeCl$ | Acetal[d] | 8 | 6.2 | 4.0 | 0.2 | 0.2 | 4.9 | 69.8 | 1.5 | >98 |
| 35 | $Co_2(CO)_8$—$Ph_3GeH$[c] | Acetal | 18 | 5.8 | 4.9 | Trace | 1.5 | 2.6 | 54.7 | 9.0 | |
| 36 | $Co_2(CO)_8$—$Ph_3GeH$[c] | Acetal[d] | 8 | 4.2 | 4.0 | 0.2 | 2.8 | 5.0 | 72.6 | 5.9 | |
| 37 | $Co_2(CO)_8$—$Ph_3GeBr$[c] | Acetal[d] | 8 | 5.6 | 3.1 | 0.2 | 2.3 | 5.0 | 68.4 | 5.8 | |
| 38 | $Co(OAc)_2$—$Ph_3GeBr$[c] | Acetal[d] | 8 | 3.9 | 3.3 | 0.2 | 2.5 | 4.0 | 69.6 | 6.9 | |
| 39 | $Co_2(CO)_8$—$Ph_3GeBr$ | Acetal[d,e] | 4 | 0.4 | | 7.1 | 6.2 | 2.7 | 77.5 | 1.6 | >98 |

[a]Run Conditions: Co, 2 mmole; Ge/Sn, 2 mmole; Acetal, 0.1 mole; EtOH, 0.3 mole; 160°C, 187.2 bars ($CO/H_2$, 1:2 molar)

[b]Abbrev. 1-Et, 2-Pro, $CH_2OEt$; 2-Et, 1-Pro, $CH_2OH$; 1-EtAcet= $CH_2OEt$
| | |
CHOH CHOEt C=0
| | |
$CH_3$ $CH_3$ $CH_3$

[c]Run conditions: 180°C, 311.3 bars ($CO/H_2$, 1:2 molar)
[d]0.05 mole, Acetal
[e]207.9 bars $CO/H_2$, 2:1 molar

Example 40

A 450 ml glass-lined pressure reactor was charged with a mixture of dicobalt octacarbonyl (2.0 mmole Co) and tetraethylgermane (2.0 mmole) in 1,1-dimethoxyethane (0.1 mole, 9.01 g) and methanol (0.3 mole, 9.60 g). The mixture was charged under a nitrogen atmosphere, the reactor sealed, flushed with $CO/H_2$ (1:1 molar), pressured to 187.2 bars with 1:1 molar syngas ($H_2CO$) and then heated to 130°C with agitation.

After 4 hours at temperature the reactor was cooled, the gas pressure noted (173.4 bars), the excess gas sampled and vented, and the red liquid product (19.6 g, 23 ml) recovered. There was no solid precipitate at this stage.

Analysis of the liquid product by glc und Karl, Fischer titration shows it to contain:

9.0% methoxyacetone
3.9% water
0.8% propylene glycol monomethyl ether
24.3% unreacted 1,1-dimethoxyethane
5.8% unreacted acetaldehyde
46.1% unreacted methanol
0.1% ethanol

Estimated conversion of 1,1-dimethoxyethane+acetaldehyde=24%. Estimated yield of methoxyacetone (basis aldehyde+acetal converted)=82%. Cobalt recovery in the product solution was >98%.

Analysis of a typical gas sample showed the presence of:

58% carbon monoxide
41% hydrogen
0.2% carbon dioxide

The methoxyacetone was recovered from the crude liquid product by fractional distillation in vacuo. Identification of the product fractions was confirmed by glc trapping and nmr analyses.

Examples 41—52

Following the same procedure as in Example 40, a number of additional Examples were carried out in which 1,1-dimethoxyethane and methanol were reacted with synthesis gas in the presence of a variety of germanium and tin promoters. Data relating to these Examples are included in Tables 6 and 7 which follow.

TABLE 6

Methoxyacetone from synthesis gas plus 1,1-dimethoxyethane[a]

| Example | Catalyst composition | liquid product composition (%)[b] MeAcet | 2-Me-1-Pro + 1-Me-2-Pro | Acetal | $CH_3CHO$ | MeOH | $H_2O$ | EtOH | Acetal conv(%) | MeAcet[c] Yield(%) | Cobalt recov.(%) |
|---|---|---|---|---|---|---|---|---|---|---|---|
| 40 | $Co_2(CO)_8$—$Et_4Ge$ | 9.0 | 0.8 | 24.3 | 5.8 | 46.1 | 3.9 | 0.1 | 24 | 82 | >98 |
| 41 | $Co_2(CO)_8$—$Ph_3GeCl$ | 6.4 | 0.2 | 29.1 | 6.0 | 47.0 | 3.6 | 0.1 | 17 | 78 | >98 |
| 42 | $Co_2(CO)_8$—$Ph_3GeBr$ | 4.7 | 0.2 | 30.6 | 6.7 | 46.3 | 4.5 | 0.1 | 10 | 95 | 27 |
| 43 | $Co_2(CO)_8$—$CsGeCl_3$ | 1.1 | 0.2 | 28.8 | 3.2 | 57.5 | 1.9 | — | 22 | 11 | >98 |
| 44 | $Co_2(CO)_8$—$Ph_4Ge$ | 7.2 | 0.2 | 24.4 | 4.7 | 46.7 | 4.4 | 0.3 | 24 | 70 | >98 |
| 45 | $Co_2(CO)_8$—$Me_3GeI$ | 2.4 | 0.2 | 32.3 | 17.7 | 20.3 | 12.6 | 0.5 | <10 | N.D.[d] | >98 |
| 46 | $Co_2(CO)_8$—$Me_3GeBr$ | 5.0 | 0.2 | 31.2 | 4.5 | 40.3 | 4.6 | 0.1 | 11.8 | >95 | >98 |
| 47 | $Co_2(CO)_8$—$Et_3GeCl$ | 7.2 | 0.3 | 27.7 | 5.4 | 45.2 | 3.9 | 0.3 | 17 | 93 | >98 |
| 48 | $Co_2(CO)_8$—$Bu_3SnH$ | 1.3 | 0.1 | 34.6 | 2.9 | 48.2 | 1.2 | 1.2 | 20 | 13 | >98 |

[a]Reaction charge; $CO_2(CO)_8$, 2.0 mmole Co; Ge/Sn, 2.0 mmole; MeOH, 0.3 mole; 1,1-dimethoxyethane, 0.1 mole.
Reaction conditions: 130°C, 187.2 bars $CO/H_2$ (1:1) initial pressure, 4 hours
[b]Abbreviations: MeAcet, $MeOCH_2CCH_3$; 1-Me-2-Pro, $MeOCH_2CHCH_3$; 1-Me-1-Pro, $HOCH_2CHCH_3$; Acetal, $(MeO)_2CHCH_3$.
||　　　　　　　　|
O　　　　　　　　OMe

[c]Methoxyacetone yield basis acetaldehyde+acetal converted
[d]N.D., Not Determined

TABLE 7

Methoxyacetone from synthesis gas plus 1,1-dimethoxyethane[a]

| Example | Catalyst composition | Operating temp. (°C) | Liquid product composition (%)[b]: MeAcet | 2-Me-1-Pro + 1-Me-2-Pro | Acetal | $CH_3CHO$ | MeOH | EtOH | Acetal conv(%) | MeAcet[c] Yield(%) |
|---|---|---|---|---|---|---|---|---|---|---|
| 49 | $Co_2(CO)_8$—$Et_3GeCl$ | 130 | 7.2 | 0.3 | 27.7 | 5.4 | 45.2 | 0.3 | 17 | 93 |
| 50 | $Co_2(CO)_8$—$Et_3GeCl$ | 145 | 7.9 | 0.4 | 19.1 | 5.5 | 46.6 | 1.0 | 30 | 62 |
| 51 | $Co_2(CO)_8$—$Et_3GeCl$ | 160 | 8.3 | 0.7 | 10.1 | 5.1 | 46.7 | 4.6 | 48 | 45 |
| 52 | $Co_2(CO)_8$—$Et_3GeCl$ | 180 | 4.1 | 6.0 | 4.6 | 3.8 | 39.3 | 8.2 | 69 | 15 |

[a]Reaction charge: $CO_2(CO)_8$, 2.0 mmole Co; Ge, 2.0 mmole; MeOH, 0.3 mole; 1,1-dimethoxyethane, 0.1 mole
Reaction conditions: 187.2 bars $CO/H_2$ (1:1) initial pressure, 4 hours

[b]Abbreviations: MeAcet, $MeOCH_2\underset{\underset{O}{\|}}{C}CH_3$; 1-Me-2-Pro, $MeOCH_2\underset{\underset{OH}{|}}{C}HCH_3$; 2-Me-1-Pro, $HO{-}CH_2\underset{\underset{OMe}{|}}{C}HCH_3$; Acetal, $(MeO)_2CHCH_3$.

[c]Methoxyacetone yield basis acetaldehyde+acetal converted

**Claims**

1. A process for preparing ethylene or propylene glycol ethers by reacting hydrogen, carbon monoxide and an aldehyde or acetal of the formula

$$RCHO \text{ or } RCH(OR')_2$$

wherein R is hydrogen or methyl and R' is $C_{1-10}$ alkyl, in the presence of a cobalt-containing catalyst and an alcohol of the formula

$$R'OH$$

wherein R' is as defined above, at a pressure of at least 35 bars and a temperature of 50 to 350°C, characterized in that the reaction is conducted in the presence of a tin-containing compound or a germanium-containing compound as catalyst promoter.

2. A process according to Claim 1 characterized in that formaldehyde or an acetal thereof or a formaldehyde-releasing compound is reacted at a temperature of from 50 to 300°C and a pressure from 70 to 350 bars to give a product containing ethylene glycol ethers.

3. A process according to Claim 1 characterized in that acetaldehyde or an acetal thereof is reacted at a temperature of 50 to 350°C and a pressure of 70 to 700 bars to give a product containing propylene glycol ether of the formulae

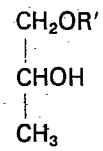

and

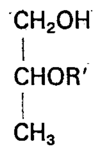

and an alkoxyacetone of the formula

$$CH_3—CO—CH_2OR'$$

wherein R' has the meaning given in Claim 1.

4. A process according to any of Claims 1 to 3 characterized in that the cobalt-containing catalyst is one or more oxides of cobalt, a cobalt salt of a mineral acid, a cobalt salt of an organic carboxylic acid or a cobalt carbonyl or hydrocarbonyl derivative.

5. A process according to Claim 4 characterized in that the cobalt-containing catalyst is cobalt oxide, cobalt chloride, cobalt iodide, cobalt nitrate, cobalt sulfate, cobalt acetate, cobalt propionate, cobalt acetylacetonate, or dicobalt octacarbonyl.

6. A process according to any of Claims 1 to 5 characterized in that the catalyst promoter is trimethyltin chloride, tributyltin chloride, tributyltin hydride, or tributyltin bromide.

7. A process according to any of Claims 1 to 5 characterized in that the catalyst promoter is triphenylgermanium chloride triphenylgermanium bromide, triphenylgermanium hydride, triethylgermanium chloride, tetraethylgermane, tetraphenylgermane or trimethylgermanium bromide.

8. A process according to any of Claims 1 to 7 characterized in that the temperature is from 100 to 250°C.

**Patentansprüche**

1. Verfahren zur Herstellung von Ethylen- oder Propylenglykoläthern durch Umsetzung von Wasserstoff, Kohlenmonoxid und einem Aldehyd oder Acetal der Formel

$$RCHO \text{ oder } RCH(OR')_2,$$

worin R Wasserstoff oder Methyl und R' $C_1—C_{10}$ Alkyl bedeuten, in Gegenwart eines Kobalt ethaltenden Katalysators und eines Alkohols der Formel

$$R'OH$$

worin R' die oben angegebene Bedeutung besitzt, bei einem Druck von mindestens 35 bar und einer Temperatur von 50 bis 350°C, dadurch gekennzeichnet, daß die Umsetzung in Gegenwart einer Zinn enthaltenden Verbindung oder einer Germanium enthaltenden Verbindung als Promoter des Katalysators durchgeführt wird.

2. Verfahren nach Anspruch 1, dadurch gekennzeichnet, daß Formaldehyd oder ein Acetal desselben oder eine Formaldehyd abgebende Verbindung bei einer Temperatur von 50 bis 300°C und einem Druck von 70 bis 350 bar unter Bildung eines Produktes umgesetzt wird, das Ethylenglykoläther enthält.

3. Verfahren nach Anspruch 1, dadurch gekennzeichnet, daß Acetaldehyd oder ein Acetal desselben bei einer Temperatur von 50 bis 350°C und einem Druck von 70 bis 700 bar unter Bildung eines Produkts umgesetzt wird, das Propylenglykoläther der Formel

$$\begin{array}{ccc} CH_2OR' & & CH_2OH \\ | & & | \\ CHOH & \text{und} & CHOR' \\ | & & | \\ CH_3 & & CH_3 \end{array}$$

und eine Alkoxyaceton der Formel

$$CH_3—CO—CH_2OR',$$

worin R' die in Anspruch 1 angegebene Bedeutung besitzt, enthält.

4. Verfahren nach einem der Ansprüche 1 bis 3, dadurch gekennzeichnet, daß als Kobalt enthaltender Katalysator ein oder mehrere Oxide des Kobalts, ein Kobaltsalz einer Mineralsäure, ein Kobaltsalz einer organischen Carbonsäure oder ein Kobaltcarbonyl- oder -hydrocarbonyl-Derivat eingesetzt wird.

5. Verfahren nach Anspruch 4, dadurch gekennzeichnet, daß als Kobalt enthaltender Katalysator Kobaltoxid, Kobaltchlorid, Kobaltjodid, Kobaltnitrat, Kobaltsulfat, Kobaltacetat, Kobaltpropionat, Kobaltacetylacetonat oder Dikobaltoctacarbonyl eingesetzt wird.

6. Verfahren nach einem der Ansprüche 1 bis 5, dadurch gekennzeichnet, daß als Katalysator-Promoter Trimethylzinnchlorid, Tributylzinnchlorid, Tributylzinnhydrid oder Tributylzinnbromid eingesetzt wird.

7. Verfahren nach einem der Ansprüche 1 bis 5, dadurch gekennzeichnet, daß als Katalysator-Promotor Triphenylgermaniumchlorid, Triphenylgermaniumbromid, Triphenylgermaniumhybrid, Triethylgermaniumchlorid, Tetraethylgerman, Tetraphenylgerman oder Trimethylgermaniumbromid eingesetzt wird.

8. Verfahren nach einem der Ansprüche 1 bis 7, dadurch gekennzeichnet, daß die Temperatur 100 bis 250°C betragt.

**Revendications**

1. Procédé de préparation d'éthers de l'éthylène ou du propylène glycol en faisant réagir de l'hydrogène, de l'oxyde de carbone et un aldéhyde ou un acétal répondant à la formule

$$RCHO \text{ ou } RCH(OR')_2$$

dans laquelle R est l'hydrogène ou un méthyle et R' est un alkyle en $C_1$ à $C_{10}$, en présence d'un catalyseur contenant du cobalt et d'un alcool répondant à la formule

$$R'OH$$

dans laquelle R' est tel que défini ci-dessus, sous une pression d'au moins $35.10^5$ Pa et une température de 50 à 350°C, caractérisé en ce que la réaction est effectuée en présence d'un composé contenant de l'étain et d'un composé contenant du germanium comme promoteur du catalyseur.

2. Procédé suivant la revendication 1, caractérisé en ce qu'on fait réagir du formaldéhyde ou un acétal de celui-ci ou un composé libérant du formaldéhyde à une température de 50 à 300°C et sous une pression de 70 à $350.10^5$ Pa, pour donner un produit contenant des éthers de l'éthylène glycol.

3. Procédé suivant la revendication 1, caractérisé en ce qu'on fait réagir de l'acétaldéhyde ou un acétal de celui-ci à une température de 50 à 350°C et sous une pression de 70 à $700.10^5$ Pa pour donner un produit contenant un éther du propylène glycol répondant aux formules

$$\begin{array}{ccc} CH_2OR' & & CH_2OH \\ | & & | \\ CHOH & \text{et} & CHOR' \\ | & & | \\ CH_3 & & CH_3 \end{array}$$

et une alkoxyacétone répondant à la formule

$$CH_3—CO—CH_2OR'$$

dans laquelle R' a la signification donnée dans la revendication 1.

4. Procédé suivant l'une quelconque des revendications 1 à 3, caractérisé en ce que le catalyseur contenant du cobalt est un ou plusieurs oxydes de cobalt, un sel de cobalt d'un acide minéral, un sel de cobalt d'un acide carboxylique organique ou un dérivé carbonylé ou hydrocarbonylé du cobalt.

5. Procédé suivant la revendication 4, caractérisé en ce que le catalyseur contenant du cobalt est de l'oxyde de cobalt, du chlorure de cobalt, de l'iodure de cobalt, du nitrate de cobalt, du sulfate de cobalt, de l'acétate de cobalt, du propionate de cobalt, de l'acétylacétonate de cobalt ou du dicobalt octacarbonyle.

6. Procédé suivant l'une quelconque des revendications 1 à 5, caractérisé en ce que le promoteur du catalyseur est du chlorure de triméthylétain, du chlorure de tributylétain, de l'hydrure de tributylétain ou du bromure de tributylétain.

7. Procédé suivant l'une quelconque des revendications 1 à 5, caractérisé en ce que le promoteur du catalyseur est du chlorure de triphénylgermanium, du bromure de triphénylgermanium, de l'hydrure de triphénylgermanium, du chlorure, de triéthylgermanium, du tétraéthylgermane, du tétraphénylgermane, ou du bromure de triméthylgermanium.

8. Procédé suivant l'une quelconque des revendications 1 à 7, caractérisé en ce que la température est de 100 à 250°C.